(19) Europäisches Patentamt
European Patent Office
Office européen des brevets

(11) EP 4 768 914 A1

(12) EUROPEAN PATENT APPLICATION

(43) Date of publication:
**01.07.2026 Bulletin 2026/27**

(21) Application number: **25223430.7**

(22) Date of filing: **15.12.2025**

(51) International Patent Classification (IPC):
***G01N 33/543*** *(2006.01)*

(52) Cooperative Patent Classification (CPC):
**G01N 33/54326; G01N 33/54393**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **31.12.2024 CN 202411999352**

(71) Applicant: **Shenzhen New Industries Biomedical Engineering Co., Ltd.**
**Shenzhen, Guangdong 518000 (CN)**

(72) Inventors:
- **ZHANG, Yun**
  **Shenzhen 518000 (CN)**
- **ZHONG, Liang**
  **Shenzhen 518000 (CN)**
- **CHEUNG, Cheuk Ling**
  **Shenzhen 518000 (CN)**
- **YAN, Lulu**
  **Shenzhen 518000 (CN)**
- **LU, Donglin**
  **Shenzhen 518000 (CN)**

(74) Representative: **Lapienis, Juozas**
**MSP Europe UAB**
**21-92 Seimyniskiu Str.**
**09236 Vilnius (LT)**

# (54) IMMUNOLOGICAL DETECTION METHOD FOR ANALYTE IN WHOLE BLOOD SAMPLE, MAGNETIC MICROSPHERE, AND MAGNETIC MICROSPHERE-PROTEIN COMPLEX

(57) The present invention provides an immunological detection method for an analyte in a whole blood sample, a magnetic microsphere, and a magnetic microsphere-protein complex. The immunological detection method includes: a) mixing and incubating the whole blood sample with an immunological detection reagent to obtain an incubation complex, wherein the immunological detection reagent includes a signal molecule and/or a magnetic microsphere-protein complex; the magnetic microsphere-protein complex includes a magnetic microsphere and a first affinity protein coated on the magnetic microsphere, the first affinity protein is a protein that can specifically bind to the analyte, and the surface of the magnetic microsphere includes methacrylate; and b) performing signal detection on the incubation complex, and obtaining the amount of the analyte based on signal intensity. The method can address the problem of low accuracy of whole blood sample detection in prior art and is suitable for the field of whole blood sample detection.

EP 4 768 914 A1

## Description

TECHNICAL FIELD

The present invention relates to the field of a whole blood sample detection, and specifically relates to an immunological detection method for an analyte in a whole blood sample, a magnetic microsphere, and a magnetic microsphere-protein complex.

BACKGROUND ART

Magnetic microspheres, due to their unique magnetic responsiveness and surface modifiability, exhibit great potential in the biomedical field, particularly in detection, such as *in vitro* diagnostics, etc. They are composed of a combination of organic polymer materials and inorganic magnetic materials, achieving rapid positioning, guidance, and separation under the influence of an external magnetic field. The surfaces of the magnetic microspheres can be chemically modified to introduce various functional groups, such as hydroxyl (-OH), carboxyl (-COOH), aldehyde (-CHO), amino ($-NH_2$), thiol (-SH), etc., which not only enhance their biocompatibility but also facilitates conjugation of subsequent antibodies or other biomolecules. The magnetic microspheres are widely used in *in vitro* diagnostic techniques such as magnetic separation immunoassays.

However, in the field of *in vitro* diagnostics, the detection of a whole blood sample is an extremely challenging task. The whole blood sample includes various components, such as red blood cells, white blood cells, and platelets, as well as various proteins and cytokines in plasma, of blood. Its complexity far exceeds that of a serum or plasma sample. When using magnetic microspheres for a whole blood detection, nonspecific adsorption between blood cells and the magnetic microspheres is a problem that cannot be ignored. Nonspecific adsorption refers to the fact that, in immunological detection, in addition to specific recognition and binding to a target antigen or antibody, the magnetic microsphere surface may also bind to non-target molecules, such as proteins, debris, etc., on blood cell surfaces, in the sample. This not only lead to an increase in a background noise of a detection signal, affecting specificity and sensitivity of the detection, but can also produce a false-positive result, and reducing accuracy of the detection.

Currently, various magnetic microsphere raw materials are available on the market for the whole blood detection, and these materials have achieved some progress in improving detection efficiency, simplifying operating procedures, etc. However, these traditional magnetic microsphere materials remain insufficient in addressing the problem of nonspecific adsorption, making them difficult to meet requirements of high sensitivity, high specificity, and high reproducibility of clinical detection. Therefore, developing a novel magnetic microsphere complex to address the problem of nonspecific adsorption in the whole blood detection has become a pressing technical challenge to be addressed in the field of current biomedical detection.

SUMMARY

The main objective of the present invention is to provide an immunological detection method for an analyte in a whole blood sample, a magnetic microsphere, and a magnetic microsphere-protein complex to solve the problem of low accuracy in a whole blood sample detection in the prior art.

To achieve the above objective, according to a first aspect of the present invention, providing an immunological detection method for the analyte in the whole blood sample, the immunological detection method includes: a) mixing and incubating the whole blood sample with an immunological detection reagent to obtain an incubation complex, wherein the immunological detection reagent includes a signal molecule and/or a magnetic microsphere-protein complex, the magnetic microsphere-protein complex includes magnetic microspheres and a first affinity protein coated on the magnetic microspheres, and the first affinity protein is a protein that specifically binds to the analyte, and the surface of the magnetic microsphere include methacrylate; and b) performing signal detection on the incubation complex, and obtaining the amount of the analyte based on signal intensity.

Further, a particle size of the magnetic microspheres is (1-8) $\mu m \pm 0.9 \mu m$.

Further, the magnetic microsphere-protein complex is obtained by coupling the magnetic microsphere with carboxyl groups as a surface group and the first affinity protein; preferably, the surface group content of the magnetic microsphere is 10-100 $\mu$mol/g; and preferably, the surface group is carboxyl group.

Further, the magnetic microsphere and the first affinity protein are coupled via a crosslinker to form the magnetic microsphere-protein complex; preferably, the crosslinker is DMTMM, and preferably, a feed ratio of DMTMM to the magnetic microsphere is (1-4):1.

Further, the surface of the magnetic microsphere is blocked with poly(bovine serum albumin) (poly(BSA)); preferably, the concentration of the poly(BSA) used for blocking is 0.3% - 2% (w/v).

Further, the a) comprises: mixing the whole blood sample with a polyethylene glycol diluent to obtain a mixed

system, and then mixing and incubating the mixed system with the immunological detection reagent to obtain the incubation complex, wherein preferably, the polyethylene glycol diluent includes a first polyethylene glycol diluent or a second polyethylene glycol diluent, and the whole blood sample includes a first whole blood sample or a second whole blood sample. Correspondingly, the a) includes a1) or a2); the a1) includes: mixing the first whole blood sample with the first polyethylene glycol diluent to obtain a first mixed system, and then mixing and incubating the first mixed system with the immunological detection reagent to obtain the incubation complex, wherein in the first mixed system, a volume percentage of the first whole blood sample is 50% - 90%, and the molecular weight of the polyethylene glycol in the first polyethylene glycol diluent is 8,000 -20,000 Da; and the a2) includes: mixing the second whole blood sample with the second polyethylene glycol diluent to obtain a second mixed system, and then mixing and incubating the second mixed system with the immunological detection reagent to obtain the incubation complex, wherein in the second mixed system, the volume percentage of the second whole blood sample is 5% - 20%, and the molecular weight of the polyethylene glycol in the second polyethylene glycol diluent is 800 - 5,000 Da.

Further, a mass content of the polyethylene glycol in the first polyethylene glycol diluent is 0.05-1 wt %; the mass content of the polyethylene glycol in the second polyethylene glycol diluent is 0.1-4 wt %; and preferably, the whole blood sample includes a fresh blood sample or an old blood sample.

Further, the immunological detection method is selected from any one of chemiluminescent immunoassay, enzyme-linked immunosorbent assay, or radioimmunoassay.

Further, the immunological detection method is the chemiluminescent immunoassay; and the signal molecule includes the second affinity protein labeled with a luminescent marker.

To achieve the above objective, according to a second aspect of the present invention, providing a magnetic microsphere, the surface of the magnetic microsphere include methacrylate, and preferably, the particle size of the magnetic microsphere is (1-8) $\mu$m $\pm$ 0.9 $\mu$m; preferably, the particle size of the magnetic microsphere is (1-2) $\mu$m $\pm$ 0.9 $\mu$m; preferably, the surface group content of the magnetic microsphere is 10-100 $\mu$mol/g; preferably, the surface group is carboxyl group; and preferably, the surface of the magnetic microsphere is blocked with poly(BSA).

To achieve the above objective, according to a third aspect of the present invention, providing a magnetic microsphere-protein complex, the magnetic microsphere-protein complex includes the above-mentioned magnetic microsphere and an affinity protein coated on the magnetic microsphere.

Applying the technical solutions of the present invention, in the immunological detection method for an analyte in a whole blood sample, a magnetic microsphere-protein complex coated with a first affinity protein is used to capture the analyte, obtaining an incubation complex, thereby achieving subsequent detection of the amount of the analyte. The surface of the magnetic microsphere used in the immunological detection method includes methacrylate, which can reduce the capture of non-analytes in the whole blood sample, effectively reducing nonspecific binding, ensuring the specificity and efficiency of immune response, and enhancing accuracy and stability of the detection signal, and providing an inventive solution for precise detection of the whole blood sample.

## DETAILED DESCRIPTION OF EMBODIMENTS

It should be noted that, unless conflicting, embodiments and features described in the embodiments of the present invention can be combined each other. The present invention will be described in detail below combined with the embodiments.

Term Explanation:

Magnetic microsphere: a magnetic microsphere is a composite microsphere prepared by combining magnetic inorganic particles (such as ferroferric oxide, $\gamma$-$Fe_2O_3$, etc.) with organic polymer materials (such as polystyrene, polyacrylic acid, etc.). They are referred to as magnetic microsphere.

As mentioned in the background, a whole blood sample detection in the prior art is affected by nonspecific adsorption, resulting in low accuracy of detection. In the present invention, the inventors unexpectedly found that the magnetic microsphere with methacrylate bound to the surface adsorb less interference components in the whole blood sample, and achieve the improvement of the accuracy and stability of the immunological detection method by the method of reducing nonspecific adsorption. Based on the discovery, the inventors developed a novel immunological detection method for an analyte in a whole blood sample, and further proposed a series of protection solutions in the present invention.

In a first typical embodiment of the present invention, providing a method for immunological detection method for an analyte in a whole blood sample, the method includes: a) mixing and incubating the whole blood sample with an immunological detection reagent to obtain an incubation complex, wherein the immunological detection reagent includes a signal molecule and/or a magnetic microsphere-protein complex, the magnetic microsphere-protein complex includes a magnetic microsphere and a first affinity protein coated on the magnetic microsphere, and the first affinity protein is a

protein that specifically binds to the analyte, and the surface of the magnetic microsphere include methacrylate; and b) performing signal detection on the incubation complex, and obtaining the amount of the analyte based on signal intensity.

In the immunological detection method, a magnetic microsphere-protein complex is provided, including: 1) the magnetic microsphere with carboxyl groups on the surface, and 2) a first affinity protein coated on the magnetic microsphere for specifically binding to the analyte. The first affinity protein includes, but is not limited to, an antibody or an antigen. By mixing and incubating the magnetic microsphere-protein complex with the whole blood sample, the incubation complex is obtained by using the specific binding of the first affinity protein to the analyte, specifically, the incubation complex includes: the magnetic microsphere, the first affinity protein, and the analyte. If the immunological detection reagent further includes a signal molecule, the signal molecule can also be bound to the incubation mixture through commonly used methods, such as specific binding, etc., in the prior art.

Further, by using the magnetic property of the magnetic microsphere to separate the incubation complex and perform signal detection, the detection of amount of the analyte can be achieved through signal intensity. The signal intensity includes but are not limited to the signal intensity of chemiluminescence, fluorescence, radioactivity, etc.

In the immunological detection method of the present invention, those skilled in the art can flexibly select any immunological detection method known in the prior art, including but not limited to methods, such as enzyme-linked immunosorbent assay (ELISA); immunofluorescence assay (IFA), radioimmunoassay (RIA), etc.

In a preferred embodiment, a particle size of the magnetic microsphere is (1-8) $\mu$m $\pm$ 0.9 $\mu$m. In a more preferred embodiment, the particle size of the magnetic microspheres is (1-2) $\mu$m $\pm$ 0.9 $\mu$m.

The magnetic microsphere with the specific particle size can provide an appropriate surface area, thereby ensuring the coating amount of the first affinity protein and improving detection efficiency, and also ensuring stability and reproducibility of signal, and are suitable for various immunological detection methods and automated detection equipment in the prior art.

An average particle size of the above-mentioned magnetic microsphere is 1um to 8um. "$\pm$ 0.9$\mu$m" represents an error of the particle size of the magnetic microsphere is $\pm$ 0.9 $\mu$m. That is, the average particle size of each magnetic microsphere fluctuates by 0.9 $\mu$m up and down. In a preferred embodiment, the magnetic microsphere-protein complex is obtained by coupling the magnetic microsphere with carboxyl groups on the surface and the first affinity protein; preferably, the surface group content of the magnetic microsphere is 10-100 $\mu$mol/g; and preferably, the surface group is carboxyl group.

A high density of the carboxyl groups can bind to affinity proteins more firmly, reduce nonspecific binding, and improve accuracy and reliability of the detection, particularly perform well in high-throughput detection.

The magnetic microsphere, serving as a core carrier, contains carboxyl (-COOH) groups on the surface, having the ability of covalently coupling with the first affinity protein, ensuring stable binding to proteins. The preferred surface group content of the magnetic microspheres is between 10 $\mu$mol/g and 100 $\mu$mol/g. A group density within the range can not only effectively load a large amount of protein, but also avoid the possible steric hindrance effect of the protein caused by overly dense group arrangement, ensuring the maximization of protein function.

In a preferred embodiment, the magnetic microsphere and the first affinity protein are coupled via a crosslinker to form the magnetic microsphere-protein complex; preferably, the crosslinker is DMTMM, and preferably, a feed ratio of DMTMM to the magnetic microsphere is (1-4):1.

The use of the crosslinker can help the groups (such as carboxyl groups) on the magnetic microsphere to better couple with the first affinity protein to form the magnetic microsphere-protein complex. Preferably, the crosslinker is DMTMM (4-(4,6-dimethoxytriazine-2-yl)-4-methylmorpholine hydrochloride, with CAS No. 3945-69-5).

The principle of using DMTMM as a crosslinker to connect the magnetic microsphere to the first affinity protein includes: first, the carboxyl group react with DMTMM to form an active ester, releasing a molecule of N-methylmorpholine (NMM). Further, the obtained active ester has high reactivity and can be subjected to nucleophilic attack from amines of the first affinity protein, alcohols, or other nucleophiles (groups), releasing a molecule of 4,6-dimethoxy-1,3,5-triazine-2-ol, forming a stable amide or carboxylic acid derivative. The reaction structure formula is shown below:

[34]

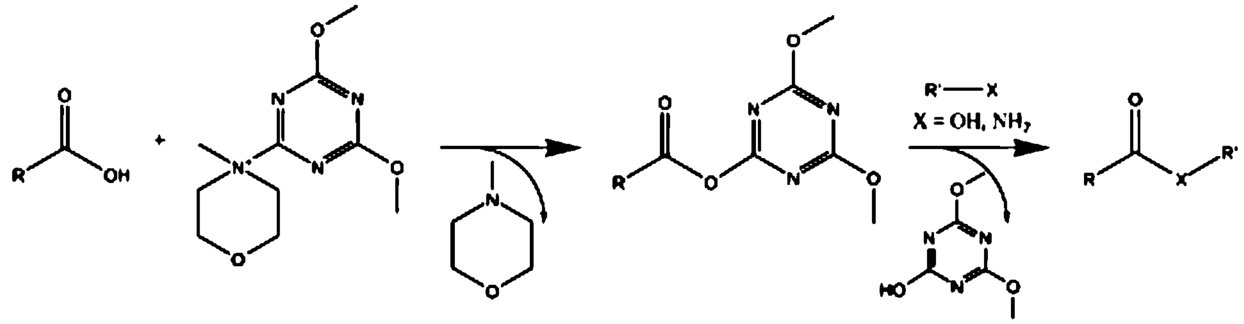


Using the crosslinker to connect the magnetic microsphere to the first affinity protein ensures uniformity and stability of coupling, while maintaining biological activity of the protein, thus avoiding nonspecific adsorption and signal loss caused by the protein shedding during detection process. By using DMTMM as the crosslinker, the magnetic microsphere-protein complex of the present invention has demonstrated significant beneficial effects in whole blood detection. The present invention effectively addresses the problems of the protein shedding and the nonspecific adsorption encountered by traditional magnetic microsphere complexes in whole blood sample detection, thereby improving sensitivity and specificity of the detection.

Preferably, when using DMTMM as the crosslinker during the reaction process, the concentration of DMTMM is preferably 3-20 mg/mL.

In a preferred embodiment, the surface of the magnetic microsphere are blocked with poly(bovine serum albumin) (poly(BSA)), Roche Catalog No. 11866737103); and preferably, the concentration of the poly(BSA) used for blocking is 0.3% - 2% (w/v).

In the present invention, poly(BSA) is preferably used for blocking treatment of the surface of the magnetic microsphere, the concentration of the poly(BSA) was controlled within the range of 0.3% to 2% (w/v). Poly(BSA), as a nonspecific protein, the blocking effect lies in shielding active sites on the magnetic microsphere surface that are not involved in antibody conjugation, thereby reducing the adsorption of blood cell components and other nonspecific molecules during subsequent detection process. Poly(BSA), with its rich amino acid sequence and excellent stability, can evenly cover the surface of the magnetic microsphere, forming a physical barrier that effectively blocks direct contact with non-target proteins, reducing the chance of nonspecific binding, thereby optimizing detection conditions and increasing the specificity of the whole blood sample detection. Compared to the magnetic microsphere blocked with BSA, the magnetic microsphere blocked with poly(BSA) have better effect in the above-mentioned immunological detection method, increasing accuracy and sensitivity of the detection, improving the repeatability of detection results, and making detection signal more stable and reliable.

Poly(BSA) (bovine serum albumin) refers to the polymeric form of bovine serum albumin. Bovine serum albumin is a kind of globulin protein including 583 amino acid residues and has a molecular weight of approximately 66.43 kDa. In biochemical experiments, BSA is widely used as a stabilizer, carrier protein, or control group of other experiments due to its rich amino acid composition and relatively stable structure. In the present invention, the term "poly" refers to a molecule formed by the combination of monomers. For BSA, a polymer means a larger molecular complex formed by multiple BSA molecules linked together by non-covalent bonds (such as hydrogen bonds, van der Waals forces, etc.). The formation of the polymer can affect its physicochemical properties and biological functions.

In a preferred embodiment, a) includes: mixing the whole blood sample with a polyethylene glycol diluent to obtain a mixed system, and then mixing and incubating the mixed system with the immunological detection reagent to obtain the incubation complex, wherein preferably, the polyethylene glycol diluent includes a first polyethylene glycol diluent or a second polyethylene glycol diluent, and the whole blood sample includes a first whole blood sample or a second whole blood sample. Correspondingly, the a) includes a1) or a2); the a1) includes: mixing the first whole blood sample with the first polyethylene glycol diluent to obtain a first mixed system, and then mixing and incubating the first mixed system with the immunological detection reagent to obtain the incubation complex, wherein in the first mixed system, a volume percentage of the first whole blood sample is 50% - 90%, (including but not limited to 50%, 55%, 60%, 65%, 70%, 75%, 80%, 85%, or 90%); and the molecular weight of the polyethylene glycol in the first polyethylene glycol diluent is 8,000 - 20,000 Da (including but not limited to 8,000Da, 9,000Da, 10,000Da, 11,000Da, 12,000Da, 13,000Da, 14,000Da, 15,000Da, 16,000Da, 17,000Da, 18,000Da, 19,000Da or 20,000Da); and the a2) includes: mixing the second whole blood sample with the second polyethylene glycol diluent to obtain a second mixed system, and then mixing and incubating the second mixed system with the immunological detection reagent to obtain the incubation complex, wherein in the second mixed system, the volume percentage of the second whole blood sample is 3% - 20% (including but not limited to

3%, 4%, 5%, 6%, 7%, 8%, 9%, 10%, 11%, 12%, 13%, 14%, 15%, 16%, 17%, 18%, 19% or 20%), and the molecular weight of the polyethylene glycol in the second polyethylene glycol diluent is 800 - 5,000 Da (including but not limited to 800 Da, 1,000 Da, 1,500 Da, 2,000 Da, 2,500 Da, 3,000 Da, 3,500 Da, 4,000 Da, 4,500 Da or 5,000 Da).

In the above-mentioned immunological detection method, preferably, before mixing the whole blood sample with the immunological detection reagent, the whole blood sample is first diluted with the polyethylene glycol diluent to obtain a mixed system, and then the mixed system is mixed and incubated with the immunological detection reagent.

Further, for items with large sample addition amount (the sample addition amount of the whole blood sample exceeds 50% of the mixed system) and for items with small sample addition amount (the sample addition amount of the whole blood sample less than 20% of the mixed system), different pre-test treatments are performed.

For items with large sample addition amount (including but not limited to high-sensitivity troponin I (hs-CtnI)), a diluent including high-molecular-weight polyethylene glycol (8,000-20,000 Da) is added to the first whole blood sample. The high-molecular-weight PEG, through its strong hydrophilicity and steric hindrance effect, can effectively promote rapid sedimentation of blood cells in reaction system, thereby reducing direct contact between blood cells and the detection reagents, significantly reducing interference of the blood cell aggregation, rupture, and nonspecific adhesion on immune response, and improving consistency of whole blood and plasma detection.

For items with small sample addition amount, (including but not limited to procalcitonin (PCT) detection), a diluent including low-molecular-weight polyethylene glycol (800-5,000 Da) is added to the second whole blood sample. The low-molecular-weight PEG, ensures the moderate dispersion of the blood cells without significantly affecting the sedimentation effect of the blood cells, avoiding the decrease in detection sensitivity caused by excessive sedimentation of the blood cells, thereby maintaining the accuracy of the test results.

In actual whole blood sample detection, components of the whole blood sample can be flexibly adjusted according to the difference of specific operation of detection items, including but not limited to diluting the whole blood sample with reagents such as polyethylene glycol, etc. The addition of the reagents does not affect the binding of the magnetic microsphere protein complex to the analyte in the present invention, nor does it affect the subsequent detection of analyte-related signals.

The first whole blood sample and the second whole blood sample in present invention are used only to distinguish the whole blood samples used in items with large sample addition amount and items with small sample addition amount. The first whole blood sample and the second whole blood sample include but are not limited to blood samples from the same individual or different individuals, or the whole blood samples collected from the same individual from the same or different batches. The first whole blood sample and the second whole blood sample are each independently selected from a fresh blood sample or an old blood sample.

In a preferred embodiment, the mass content of polyethylene glycol in the first polyethylene glycol diluent is 0.05-1wt%; the mass content of polyethylene glycol in the second polyethylene glycol diluent is 0.1-4wt%; and preferably, the whole blood sample includes the fresh blood sample or the old blood sample.

The fresh blood sample (referred to as a fresh sample for short) refers to the whole blood sample that has been stored for no more than 8 hours after blood collection, and the old blood sample (referred to an old sample for short) refer to the whole blood sample that has been stored at 2-8°C for more than 5 days. The above-mentioned immunological detection method can be used to conduct rapid detection on both the fresh sample and the old sample, and detection results are accurate and reliable.

The above-mentioned immunological detection method can achieve accurate detection of the old blood sample, facilitating to achieve accurate detection of the old blood sample in fields, such as retrospective studies, forensic identification, stored blood quality detection, drug research, genetics research, etc., avoiding the limitations on detections caused by must-use of the fresh blood sample for detecting.

In a preferred embodiment, the immunological detection method is selected from any one of a chemiluminescent immunoassay, an enzyme-linked immunosorbent assay, and a radioimmunoassay.

In a preferred embodiment, the immunological detection method is a chemiluminescent immunoassay; and the signal molecule includes a second affinity protein labeled with a luminescent marker.

Preferably, the above-mentioned second affinity protein includes, but is not limited to, an antigen and/or an antibody, and the second affinity protein is attached to the incubation complex by binding to, but is not limited to, the magnetic microsphere, the first affinity protein, etc. The luminescent marker achieves generation of a light signal during subsequent signal detection of the incubation mixture.

In a preferred embodiment, the polyethylene glycol diluent further includes, but is not limited to, any one or more of the following: a pH buffer, NaCl, bovine serum albumin, glycine, or a preservative; and preferably, the pH buffer includes one or more of potassium dihydrogen phosphate, dipotassium hydrogen phosphate, or Tris-HCl.

In a preferred embodiment, the luminescent marker includes, but is not limited to, any one or more of the following: isoluminol and its derivatives, an acridinium ester and its derivatives, or ruthenium terpyridine.

In the above-mentioned immunological detection method for the analyte in the whole blood sample, those skilled in the art can flexibly select detection methods, detection reagents, detection parameters, etc. known in the prior art to

conduct tests, all of which can achieve the immunological detection, realize the detection of the analyte in the whole blood sample, and significantly improve the detection efficiency and accuracy of the components to be detected in the whole blood sample.

In a second typical embodiment of the present invention, the magnetic microsphere is provided, wherein the surface of the magnetic microsphere includes methacrylate. Preferably, the particle size of the magnetic microsphere is (1-8) $\mu$m $\pm$ 0.9 $\mu$m. Preferably, the surface group content of the magnetic microsphere is 10-100 $\mu$mol/g. Preferably, the surface group is carboxyl group. Preferably, the surfaces of the magnetic microspheres are blocked with poly(BSA).

In a third typical embodiment of the present invention, a magnetic microsphere-protein complex is provided, wherein the magnetic microsphere-protein complex includes the magnetic microsphere and an affinity protein coated on the magnetic microsphere.

The affinity protein includes, but is not limited to, the first affinity protein capable of specifically binding to the analyte.

The above-mentioned magnetic microsphere and/or the magnetic microsphere-protein complex can be used for the immunological detection of the analyte in the whole blood sample, or for preparing relevant reagents for the immunological detection of the whole blood sample. By using the above-mentioned magnetic microsphere and/or magnetic microsphere protein complex, it can achieve accurate detection of the analyte in the whole blood sample, reducing detection bias and even detection errors caused by nonspecific adsorption of the magnetic microsphere and interfering components in the whole blood sample.

The beneficial effects of the present invention will be further explained in detail below with reference to specific embodiments.

Embodiment 1 Preparation of magnetic microspheres

1. Preparation method of oleic acid-coated magnetic microparticle:

Oleic acid-coated magnetic microparticles are prepared using a coprecipitation method. Under a condition of continuous nitrogen purging, 23.5 g of $FeCl_3 \cdot 6H_2O$ and 8.6 g of $FeCl_2 \cdot 4H_2O$ were dissolved in 500 mL of deionized water. Solution temperature was maintained at 85°C, and 27.8 mL of $NH_4OH$ was quickly added. Oleic acid was then gradually added dropwise within 10 min. The stirring speed was controlled at 600 rpm until blocky magnetite ($Fe_3O_4$) gel formed. The obtained magnetite gel was used as an aggregate of OMP, cooled to room temperature, washed 8 times with deionized water, and stored under vacuum after removing supernatant.

2. Preparation method of magnetic microspheres 0

1. 2 g of benzoyl peroxide, 95 mL of styrene, 9 mL of divinylbenzene, 33 mL of acrylic acid, and 20 g of oleic acid-coated magnetic microparticles were mixed to form an organic phase, stirring vigorously and ultrasonic treating for 10 minutes to ensure uniform dispersion of the oleic acid-coated magnetic microparticles.

2. An aqueous phase consists of 25 g of polyvinyl alcohol-1788, 30 g of sodium chloride, and 1000 mL of deionized water. The aqueous phase was added to the organic phase and the two phases were mixed at a stirring speed of 600 rpm and continuous nitrogen purging. Temperature of synthesis solution was maintained at 80°C for 15 h.

3. After synthesis process is completed, the magnetic microspheres 0 are separated from the solution using a magnet and washed 5 times with deionized water to remove stabilizers and other impurities adhering to particle surfaces.

3. Preparation method of magnetic microspheres 1

1. 3 g of benzoyl peroxide, 36 mL of methyl methacrylate, 20 mL of styrene, 8 mL of divinylbenzene, 38 mL of glycidyl methacrylate, 30 mL of cyclohexane, 16 mL of acrylic acid, and 20 g of the oleic acid-coated magnetic microparticles prepared above were mixed to form an organic phase, stirring vigorously and ultrasonic treating for 10 minutes to ensure uniform dispersion of the oleic acid-coated magnetic microparticles.

2. The aqueous phase consists of 10 g of polyvinyl alcohol-1788, 15 g of sodium chloride, and 1000 mL of deionized water. The aqueous phase was added to the organic phase and the two phases were mixed at a stirring speed of 600

rpm and continuous nitrogen purging. The temperature of the synthesis solution was raised from 45°C to 55°C within 1 h, then maintained at 60°C for 2 h, and finally maintained at 70°C and 80°C for 1 h, respectively.

3. After the synthesis process is completed, the magnetic microspheres 1 are separated from the solution using the magnet and washed 5 times with deionized water to remove stabilizers and other impurities adhering to the particle surfaces.

4. Preparation method of magnetic microspheres 2

1. 3 g of benzoyl peroxide, 32 mL of methyl methacrylate, 18 mL of styrene, 6 mL of divinylbenzene, 45 mL of glycidyl methacrylate, 30 mL of cyclohexane, 15 mL of acrylic acid, and 20 g of the oleic acid-coated magnetic microparticles prepared above were mixed to form an organic phase, stirring vigorously and ultrasonic treating for 10 minutes to ensure uniform dispersion of the oleic acid-coated magnetic microparticles.

2. The aqueous phase consists of 10 g of polyvinyl alcohol-1788, 15 g of sodium chloride, and 1000 mL of deionized water. The aqueous phase was added to the organic phase and the two phases were mixed at a stirring speed of 600 rpm and continuous nitrogen purging. The temperature of the synthesis solution was raised from 45°C to 55°C within 1 h, then maintained at 60°C for 2 h, and finally maintained at 70°C and 80°C for 1 h, respectively.

3. After the synthesis process is completed, the magnetic microspheres 2 are separated from the solution using the magnet and washed 5 times with deionized water to remove stabilizers and other impurities adhering to the particle surfaces.

5. Preparation method of magnetic microspheres 3

1. 3 g of benzoyl peroxide, 40 mL of methyl methacrylate, 18 mL of styrene, 8 mL of divinylbenzene, 30 mL of glycidyl methacrylate, 30 mL of cyclohexane, 13 mL of acrylic acid, and 20 g of the oleic acid-coated magnetic microparticles prepared above were mixed to form an organic phase, stirring vigorously and ultrasonic treating for 10 minutes to ensure uniform dispersion of the oleic acid-coated magnetic microparticles.

2. The aqueous phase consists of 10 g of polyvinyl alcohol-1788, 15 g of sodium chloride, and 1000 mL of deionized water. The aqueous phase was added to the organic phase and the two phases were mixed at a stirring speed of 600 rpm and continuous nitrogen purging. The temperature of the synthesis solution was raised from 45°C to 55°C within 1 h, then maintained at 60°C for 2 h, and finally maintained at 70°C and 80°C for 1 h, respectively.

3. After the synthesis process is completed, the magnetic microspheres 3 are separated from the solution using the magnet and washed 5 times with deionized water to remove stabilizers and other impurities adhering to the particle surfaces.

Embodiment 2 Detection method of the magnetic microspheres

1. Detection method of particle size

The magnetic microspheres to be detected were mixed thoroughly by ultrasonication for 5 min; 1.5 mL of the magnetic microspheres were transferred to a 20 mL vial, 0.2 mL of Tween is added to the vial, mixing thoroughly by ultrasonication at 60% power for 1 min. Detection was performed by using a Malvern particle size analyzer.

2. Detection method of carboxyl content

The method includes:

1) mixing thoroughly the magnetic microspheres to be tested by ultrasonication for 5 min;

2) taking 0.5 g of magnetic microsphere solution to a 250 mL beaker, dropwise adding 3 mL of 2 M hydrochloric acid

solution to the beaker, adding 150 mL of purified water, and mixing thoroughly by ultrasonication at 100% power for 1 min; sedimenting on a magnet with a magnetic field of 300-500 millitesla (mT) for 30 min, and removing supernatant;

3) adding another 150 mL of purified water and repeating the rinse twice;

4) adding 200 mL of purified water to the beaker and mixing thoroughly by ultrasonication for 1 min; and

5) detecting using a pH titrator.

Parameters of the four magnetic microspheres were obtained by the above detection method, as shown in Table 1 below:

Table 1

| | Carboxyl content/(μmol/g) | Particle size (μm) Dv50-Dv90 |
|---|---|---|
| Magnetic microsphere 0 | 463 | 1.72-8.64 |
| Magnetic microsphere 1 | 83 | 1.21-2.18 |
| Magnetic microsphere 2 | 59 | 1.32-2.94 |
| Magnetic microsphere 3 | 78 | 1.13-2.9 |

Embodiment 3

1. Preparation of magnetic microsphere complex:

The above-mentioned 20 mg of the magnetic microspheres were mixed with 5 mL of phosphate buffer solution with a pH of 5.0 and stirred for 2-3 min. After adsorbing the magnetic microspheres by an external magnetic field, the supernatant was removed. The washing step was repeated three times. After washing, the magnetic microspheres were resuspended to 20 mg/mL by adding phosphate buffer solution with a pH of 5.0. The crosslinker and the blocking agent were added to the suspension at the concentrations shown in Table 2 and mixed thoroughly. Finally, the magnetic microspheres were mixed thoroughly with the antibody at a mass ratio of 1 mg:10 μg (self-produced antibody by NEW INDUSTRIES BIOMEDICAL ENGINEERING CO., LTD.). The mixture was place in a constant temperature shaker at 40°C for shaking reaction for 2 h. After the reaction is completed, the magnetic microspheres were magnetically separated and washed three times with buffer solution, then resuspended in magnetic microsphere diluent to 20 mg/mL for storage.

Table 2

| Magnetic microsphere complex | Types of magnetic microspheres | Crosslinker | Concentration of crosslinker | Blocking agent | Concentration of blocking agent |
|---|---|---|---|---|---|
| Control group | Magnetic microsphere 0 | CDC | 5mg/mL | BSA | 0.5wt% |
| Experimental group 1 | Magnetic microsphere 1 | CDC | 5mg/mL | BSA | 0.5 wt % |
| Experimental group 2 | Magnetic microsphere 2 | CDC | 5mg/mL | BSA | 0.5 wt % |
| Experimental group 3 | Magnetic microsphere 3 | CDC | 5mg/mL | BSA | 0.5 wt % |
| Experimental group 4 | Magnetic microsphere 1 | DMTMM | 5mg/mL | BSA | 0.5 wt % |
| Experimental group 5 | Magnetic microsphere 1 | DMTMM | 10mg/mL | BSA | 0.5 wt % |
| Experimental group 6 | Magnetic microsphere 1 | DMTMM | 15mg/mL | BSA | 0.5 wt % |
| Experimental group 7 | Magnetic microsphere 1 | DMTMM | 20mg/mL | BSA | 0.5 wt % |
| Experimental group 8 | Magnetic microsphere 1 | DMTMM | 15mg/mL | Poly(BSA) | 0.5 wt % |
| Experimental group 9 | Magnetic microsphere 1 | DMTMM | 15mg/mL | / | / |

The magnetic microsphere complex prepared as prepared above was stored in a diluent, wherein the components of the diluent adopt liquid components of the magnetic microsphere preservation solution in the PCT kit produced by

New Industry Biotechnology. The liquid components include buffer substances: 0.04% potassium dihydrogen phosphate, 0.4% disodium hydrogen phosphate, 0.8% NaCl, 0.5% bovine serum albumin, 1M glycine, 0.5 mg/mL goat anti-human IgG, and preservative Proclin300 0.15%.

2. Preparation of samples

A fresh normal human whole blood sample was collected, labeled "sample", and the sample was divided into three blood collection tubes: one tube remained untreated and was labeled as "sample F"; one tube was subjected to ultrasonic disruption treatment for 20 min and was labeled as "sample S"; and the other tube was centrifuged to obtain plasma for later use and was labeled as "sample P".

The above steps were repeated to collect 9 groups of samples for later use.

3. Detection of sample F and sample S

The detection was performed using the PCT kit independently produced by New Industry Biotechnology. The magnetic microsphere reagent was replaced with the magnetic microsphere reagent prepared in experimental group in step 1. The sample diluent and marker components in the kit were used for detection in a chemiluminescence immunoassay analyzer MAGLUMI X3 independently produced by New Industry Biotechnology. The detection principle was: chemiluminescence immuno-sandwich method.

One PCT antibody (capture antibody) was used to coat the magnetic microspheres, and another PCT antibody (detection antibody) is labeled with ABEI (luminescent agent, N-(4-aminobutyl)-N-ethylisoluminol). The sample, luminescent marker, buffer, and magnetic microspheres were mixed and incubated together. The analyte in the sample forms an immune complex with the ABEI-labeled PCT antibody and the PCT antibody on the magnetic microspheres. After incubation, unbound substances were removed by magnetic separation and washing. Finally, substrate solution for the fully automated immunoassay system is added to initiate chemiluminescent reaction, generating a light signal. The relative light unit (RLU) measured by a photomultiplier tube has a certain proportional relationship with PCT concentration in the sample.

The detection steps are as follows. 20 μL of sample was added to a reaction cup, then 20 μL of the magnetic microsphere component, 50 μL of buffer, and 50 μL of the luminescent marker working solution was added to the reaction cup and mixed thoroughly. After mixing thoroughly, the mixture was incubated at 37°C for 14 min to allow the target analyte in the sample to be detected to form a double-antibody sandwiched ternary immune complex with a specific antibody coated with the magnetic microspheres and another specific antibody labeled with ABEI in the first reagent. After incubation, unbound substances were removed by magnetic separation and washing. Finally, hydrogen peroxide and sodium hydroxide were added to the system to excite the marker to emit light, and the light signal is detected to obtain the RLU value.

The detection results are shown in Tables 3 and Table 4 below, wherein the control group and experimental groups 1-4 represent the whole blood sample detection using the magnetic microspheres prepared in Table 2 above.

Table 3 PCT detection deviation results of sample F and sample S

| Sample | Control group | Experimental group 1 | Experimental group 2 | Experimental group 3 | Experimental group 4 |
|---|---|---|---|---|---|
| No. | Deviation | Deviation | Deviation | Deviation | Deviation |
| Mean absolute deviation | 122% | 6.49% | 7.23% | 9.14% | 4.55% |
| Sample | Experimental group 5 | Experimental group 6 | Experimental group 7 | Experimental group 8 | Experimental group 9 |
| No. | Deviation | Deviation | Deviation | Deviation | Deviation |
| Mean absolute deviation | 4.93% | 3.43% | 3.29% | 2.34% | 2.95% |

In Table 3, Deviation = (Sample S RLU - Sample F RLU) / Sample F RLU × 100%.

The detection was repeated 10 times for each sample, and the CV value was calculated. The results are shown in Table 4 below.

Table 4: Detection repeatability verification of Sample F and Sample S

| Sample F | Control group | Experimental group 6 | Experimental group 8 | Experimental group 9 |
|---|---|---|---|---|
| Average CV (%) | 11.34 | 4.26 | 1.91 | 3.24 |
| Sample S | Control group | Experimental group 6 | Experimental group 8 | Experimental group 9 |
| Average CV (%) | 19.42 | 6.57 | 2.99 | 7.37 |

The results showed that when the magnetic microspheres in the experimental group were used to detect sample F, the RLU of the whole blood sample of normal people was significantly reduced compared with 122% of the control group, and the RLU deviations of detecting sample S (with more cell debris) and sample F were all within 10%, indicating that the magnetic microspheres upgraded by the present solution can reduce the interference of blood cell debris on the immune response, and further using DMTMM as a crosslinker can reduce the non-specific adsorption of the magnetic microsphere complex in the whole blood sample reaction system; further, the RLU deviation of detecting sample S and sample F in experimental group 8 was only 2.34%, indicating that the combined use of the DMTMM as a crosslinker and the poly(BSA) as a blocking agent can further improve the whole blood anti-interference ability of the modified magnetic microspheres; and the deviations of experimental group 9 and experimental group 6 are not much different, indicating that using DMTMM alone also has a good effect. It is speculated that DMTMM, in addition to serving as a crosslinker, also has a certain blocking effect. According to the results of the repeatability of the whole blood sample detection in Table 4, the repeatability CV of the sample S in experimental groups 6, 8, and 9 are all within 8%. The above results comprehensively indicate that using the present solution to modify the magnetic microspheres and further treat the magnetic microspheres with the crosslinker of DMTMM or the blocking agent of poly(BSA) can greatly reduce the nonspecific adsorption level of the whole blood sample and improve the accuracy of the whole blood detection.

In the sample S obtained through ultrasonic disruption treatment, the whole blood sample was subjected to ultrasonic disruption to simulate a detection environment where contents were released caused by blood cell rupture in extreme conditions. Using magnetic microspheres in the prior art for detection, it was found that the light intensity deviation between broken blood samples and normal blood samples could reach up to 122% at most. However, when using the magnetic microspheres in the present invention, the deviation between the two samples was significantly reduced, demonstrating a strong ability to resistance to nonspecific adsorption of the magnetic microspheres in the present invention. Using DMTMM as a crosslinker can further reduce the nonspecific adsorption of the magnetic microsphere complex, and the deviation is further reduced after blocking with poly(BSA).

4. Detection of sample F and sample P

Plasma sample values were detected using a Roche immunoassay analyzer and the accompanying PCT chemiluminescence kit (Cat. No. (240)09318712190) and compared with the detection results of sample F in the present invention, wherein the whole blood sample was detected by HCT, and the detection results were converted to concentration values of target substance in plasma and then the absolute deviation was calculated with the Roche detection results.

$$\text{Plasma concentration} = \text{whole blood detection concentration} / (1\text{-HCT});$$

HCT was detected by spectrophotometry and the calculation formula was:

$$abs = log_{10}\frac{\text{Cuvette-Free}\,\mathrm{RLU}_{\text{Every experiment}}}{\text{Whole Blood}\,\mathrm{RLU}_{\text{Every sample}}} - log\,10\,\frac{\text{Cuvette-Free}\,\mathrm{RLU}_{\text{Per kit}}}{\text{Empty Cuvette}\,\mathrm{RLU}_{\text{Per kit}}}$$

Absolute deviation = |Plasma sample detection value - whole blood sample detection value| / Plasma sample detection value.

The detection results are shown in Table 5.

Table 5:

| Sample | Control group | Experimental group 1 | Experimental group 2 | Experimental group 3 | Experimental group 4 |
|---|---|---|---|---|---|
| Mean absolute deviation | 20.3% | 7.8% | 8.65% | 8.96% | 4.98% |
| Sample | Experimental group 5 | Experimental group 6 | Experimental group 7 | Experimental group 8 | Experimental group 9 |
| Mean absolute deviation | 4.56% | 3.89% | 4.12% | 2.34% | 3.66% |

The results indicate that the magnetic microsphere complex prepared by the present solution can significantly improve the consistency of detection results of the whole blood and the plasma. The deviation between the magnetic microsphere complex prepared by DMTMM as a crosslinker and the Roche plasma detection results is less than 5%, demonstrating high accuracy.

Embodiment 4

1. Preparation of sample diluent

The sample diluent components of experimental group 9 include 50 mM Tris buffer pairs, 0.2% NaCl, 0.5% bovine serum albumin, and 0.15% Proclin 300 preservative. Further, PEG with different molecular weights was added according to the concentrations shown in experimental groups 10-15 to verify the deviation of fresh blood samples and old blood samples and plasma.

Experimental group 10: PEG with a MW (molecular weight) of 800 was added to the sample diluents from experimental group 9 to a final PEG concentration of 0.1 wt%.

Experimental group 11: PEG with a MW (molecular weight) of 4000 was added to the sample diluents from experimental group 9 to a final PEG concentration of 0.1 wt%.

Experimental Group 12: PEG with a MW (molecular weight) of 5000 was added to the sample diluents from experimental group 9 to a final PEG concentration of 0.1 wt%.

Experimental Group 13: PEG with a MW (molecular weight) of 4000 was added to the sample diluents from experimental group 9 to a final PEG concentration of 2 wt%.

Experimental Group 14: PEG with a MW (molecular weight) of 4000 was added to the sample diluents from experimental group 9 to a final PEG concentration of 4 wt%.

2. Preparation of sample

One fresh whole blood sample (sample F) was collected. Each sample F was divided into two blood collection tubes. One tube was centrifuged to obtain plasma for later use and the PCT concentration in the plasma was detected. The other tube was left untreated and the PCT concentration in the whole blood was detected.

Another old sample (sample S) that has been placed for 5 days was collected and also divided into two blood collection tubes. Plasma was obtained by centrifugation according to the above step for later use. The other tube was left untreated and was directly detected.

Repeat the above steps to collect 9 groups of samples for later use.

3. Detection of sample

The values of PCT in the plasma and whole blood of above samples were detected according to the steps in Embodiment 3, and the deviations were calculated. The results are shown in Table 6 below.

Table 6

| Sample F | Experiment al group 9 | Experiment al group 10 | Experiment al group 11 | Experimental group 12 | Experiment al group 13 | Experiment al group 14 |
|---|---|---|---|---|---|---|
| Mean ab- solute de- viation | 3.76% | 3.54% | 3.21% | 3.09% | 2.85% | 3.16% |

(continued)

| Sample S | Experiment al group 9 | Experiment al group 10 | Experiment al group 11 | Experimental group 12 | Experiment al group 14 | Experiment al group 15 |
|---|---|---|---|---|---|---|
| Mean ab-solute de-viation | 5.97% | 5.21% | 4.96% | 4.87% | 4.21% | 4.36% |

The results indicate that when detecting items with sample addition amount of whole blood less than 20% of the volume ratio of the detection system, adding polyethylene glycol with a molecular weight of 800-5,000 Da to the sample diluent can further effectively reduce the detection deviation between the fresh whole blood samples and the old whole blood samples and the plasma.

Embodiment 5

The detection was performed using a chemiluminescence immunoassay analyzer Maglumi X3 independently produced by New Industry Biotechnology and a matching self-produced luminescence kit hs-cTnI. The sample diluent in the kit was replaced with a diluent supplemented with polyethylene glycol diluent, and the magnetic microspheres and protein complex in the first reagent were replaced by those prepared and obtained according to experimental group 9 in Embodiment 3. The consistency of whole blood and plasma samples were detected, and the repeatability is also detected. The detection steps were as follows:

1. Preparation of diluent:

Control group 2: basic diluent with components consisting of 50 mM Tris buffer pairs, 0.2% NaCl, 0.5% bovine serum albumin, and 0.015% Proclin300 preservative.
Experimental group 16: adding PEG with a MW (molecular weight, Da) of 8,000 to the base diluent of control group 2 to a final PEG concentration of 0.1 wt%.
Experimental group 17: adding PEG with a MW of 20,000 to the base diluent of control group 2 to a final PEG concentration of 0.1 wt%.
Experimental group 18: adding PEG with a MW of 10,000 to the base diluent of control group 2 to a final PEG concentration of 0.05 wt%.
Experimental group 19: add PEG with a MW of 10,000 to the base diluent of control group 2 to a final PEG concentration of 1 wt%.

2. Preparation of sample:

One fresh whole blood sample (sample F) was collected. Each sample F is divided into two blood collection tubes. One tube is centrifuged (3,000 rpm, 5 min) to obtain plasma for later use and hs-cTnI concentration value in the plasma was detected. The other tube is left untreated and the hs-cTnI concentration value in the whole blood was detected.
Another old sample (sample S) that has been placed for 5 days was collected and also divided into two blood collection tubes. Plasma was obtained by centrifugation according to the above step for later use. The other tube was left untreated and was directly detected.

Repeat the above steps to collect 9 groups of samples for later use.

3. Detection reagents

In addition to the sample diluent, other components of the hs-cTnI Luminescence Kit (Cat. No. 130256014M) produced by NEW INDUSTRIES BIOMEDICAL ENGINEERING CO., LTD. were used for the detection of the above-mentioned samples. The other components include the following.
First Reagent: magnetic microspheres coated with an antibody that specifically binds to hs-cTnI. The preparation method and coating protocol for the magnetic microspheres follow the method described in experimental group 9. The liquid components include 0.04% potassium dihydrogen phosphate, 0.4% dipotassium hydrogen phosphate, 0.8% NaCl, 0.5% bovine serum albumin, and 0.15% Proclin300 preservative.
Second Reagent: marker reagent of another ABEI-labeled specific antibody that specifically binds to hs-cTnI. The reagent includes 70mM Tris buffer pairs, 0.4% NaCl, 0.5% bovine serum albumin, 1M glycine, and 0.15% Proclin300

preservative.

4. Detection of sample

Whole blood samples were detected using a chemiluminescence immunoassay analyzer Maglumi X3 from New Industry Biotechnology. The steps are as follows.

The whole blood sample of step 2 was detected using the sample diluents prepared for the control group and experimental groups and the first and the second reagents produced by NEW INDUSTRIES BIOMEDICAL ENGINEERING CO., LTD. 150 μL of sample was added to the reaction cup and the reagent sampling was performed in the following order: the first reagent, the diluent of the experimental group in step 1, and the second reagent. After sampling, all were added to the reaction cup and mixed thoroughly. After mixing thoroughly, the mixture was incubated at 37°C for 14 min, enabling to form a double-antibody sandwiched ternary immune complex between the target analyte in the sample to be detected and a magnetic microspheres-coated specific antibody and another ABEI-labeled specific antibody in the first reagent. After incubation, unbound substances were removed by magnetic separation and washing. Finally, hydrogen peroxide and sodium hydroxide, substrate solutions used in the automated immunoassay system, were added to initiate the chemiluminescent reaction, generating a light signal. The relative light unit (RLU) was measured by a photomultiplier tube. The above steps were repeated for the whole blood sample detections to obtain RLU values, calculating the concentration values of the target substance hs-cTnI, in the samples, and converting the detection results into the concentration values of the target substance in the plasma through HCT detection. The conversion formula is the same as in the above-mentioned embodiment.

The above-mentioned plasma samples were detected using a Beckman analyzer Access2 81600N and the accompanying reagent hs-cTnI (REF: B52699), to determine the concentration of the target substance in the plasma.

Based on the detection results, the absolute deviations of the detections of 9 groups whole blood samples and plasma samples were calculated using the same method as in the above-mentioned embodiments.

The detection results are shown in Table 7 below.

Table 7

| Sample F | Control group 2 | Experimental group 16 | Experimental group 17 | Experimental group 18 | Experimental group 19 |
|---|---|---|---|---|---|
| Mean absolute deviation | 3.15% | 2.96% | 3.11% | 2.41% | 3.09% |
| Sample S | Control group 2 | Experimental group 10 | Experimental group 11 | Experimental group 12 | Experimental group 14 |
| Mean absolute deviation | 4.99% | 4.85% | 3.97% | 3.15% | 4.21% |

The results indicate that when detecting items with sample addition amount of whole blood exceeds 50% of the volume ratio of the detection system, adding polyethylene glycol with a molecular weight of 8,000-20,000 Da to the sample diluent can further effectively reduce the detection deviation between the fresh whole blood samples and the old whole blood samples and the plasma.

From the above description, it can be seen that the above-mentioned embodiments of the present invention achieve the following technical effects: the immunological detection method and magnetic microsphere-protein complex provided by the present invention improve the sensitivity and specificity of detection by utilizing magnetic microspheres with methacrylate on the surface. The specific particle size and surface group content of the magnetic microspheres, as well as surface-blocked poly(BSA), can further effectively reduce nonspecific binding (adsorption), enhancing the stability of the detection signal. In addition, by adjusting the composition of the polyethylene glycol diluent, the pretreatment of the whole blood samples can be optimized, further improving the efficiency and accuracy of the detection, and it is applicable to various detection methods and detection solutions in the prior art. The present solution is particularly suitable for rapid and accurate detection of the fresh or old blood samples, providing a powerful tool for clinical diagnosis and disease monitoring.

The above description is merely preferred embodiments of the present invention and is not intended to limit the present invention. For those skilled in the art, the present invention can have various modifications and variations. Any modifications, equivalent substitutions, improvements, etc. made within the spirit and principles of the present invention are intended to be included within the scope of protection of the present invention.

## Claims

1. An immunological detection method for an analyte in a whole blood sample, **characterized in that** the immunological detection method comprises:

    a) mixing and incubating the whole blood sample with an immunological detection reagent to obtain an incubation complex,

        wherein the immunological detection reagent comprises a signal molecule and/or a magnetic microsphere-protein complex,
        the magnetic microsphere-protein complex comprises a magnetic microsphere and a first affinity protein coated on the magnetic microsphere, and
        the first affinity protein is a protein that specifically binds to the analyte, and a surface of the magnetic microsphere comprises methacrylate; and

    b) performing a signal detection on the incubation complex, and obtaining an amount of the analyte based on a signal intensity.

2. The immunological detection method according to claim 1, wherein a particle size of the magnetic microsphere is (1-8) $\mu m \pm 0.9 \mu m$.

3. The immunological detection method according to claim 1, wherein the magnetic microsphere-protein complex is obtained by coupling the magnetic microsphere with carboxyl groups as surface groups and the first affinity protein;

    preferably, a content of the surface groups of the magnetic microsphere is 10 μmol/g - 100 μmol/g; and preferably, the surface groups are the carboxyl groups.

4. The immunological detection method according to any one of claims 1-3, wherein the magnetic microsphere and the first affinity protein are coupled via a crosslinker to form the magnetic microsphere-protein complex; and preferably, the crosslinker is DMTMM.

5. The immunological detection method according to any one of claims 1-4, wherein the surface of the magnetic microsphere is blocked with poly(BSA).

6. The immunological detection method according to any one of claims 1-5, wherein the a) comprises: mixing the whole blood sample with a polyethylene glycol diluent to obtain a mixed system, and then mixing and incubating the mixed system with the immunological detection reagent to obtain the incubation complex;

    preferably, the polyethylene glycol diluent comprises a first polyethylene glycol diluent or a second polyethylene glycol diluent, and the whole blood sample comprises a first whole blood sample or a second whole blood sample, wherein the a) comprises a1) or a2);
    the a1) comprises:

        mixing the first whole blood sample with the first polyethylene glycol diluent to obtain a first mixed system, and then mixing and incubating the first mixed system with the immunological detection reagent to obtain the incubation complex, wherein
        in the first mixed system, a volume percentage of the first whole blood sample is 50% - 90%, and a molecular weight of polyethylene glycol in the first polyethylene glycol diluent is 8,000 - 20,000 Da; and
        the a2) comprises:

            mixing the second whole blood sample with the second polyethylene glycol diluent to obtain a second mixed system, and then mixing and incubating the second mixed system with the immunological detection reagent to obtain the incubation complex, wherein
            in the second mixed system, a volume percentage of the second whole blood sample is 5% - 20%, and a molecular weight of polyethylene glycol in the second polyethylene glycol diluent is 800 - 5,000 Da.

7. The immunological detection method according to claim 6, wherein

a mass content of the polyethylene glycol in the first polyethylene glycol diluent is 0.05-1 wt %;
a mass content of the polyethylene glycol in the second polyethylene glycol diluent is 0.1-4 wt %; and
preferably, the whole blood sample comprises a fresh blood sample or an old blood sample.

**8.** The immunological detection method according to any one of claims 1-7, wherein the immunological detection method is selected from any one of chemiluminescent immunoassay, enzyme-linked immunosorbent assay, or radioimmunoassay.

**9.** The immunological detection method according to claim 8, wherein the immunological detection method is the chemiluminescent immunoassay; and
the signal molecule comprises a second affinity protein labeled with a luminescent marker.

**10.** A magnetic microsphere, **characterized in that** a surface of the magnetic microsphere comprises methacrylate, and

preferably, a particle size of the magnetic microsphere is (1-8) μm ± 0.9 μm;
preferably, a content of surface groups of the magnetic microsphere is 10 μmol/g - 100 μmol/g;
preferably, the surface groups are carboxyl groups; and
preferably, the surface of the magnetic microsphere is blocked with poly(BSA).

**11.** A magnetic microsphere-protein complex, **characterized in that** the magnetic microsphere-protein complex comprises the magnetic microsphere according to claim 10 and an affinity protein coated on the magnetic microsphere.

Europäisches Patentamt
European Patent Office
Office européen des brevets

# EUROPEAN SEARCH REPORT

Application Number
EP 25 22 3430

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | Zuzana Svobodova ET AL: "ON-CHIP ELISA ON MAGNETIC PARTICLES: ISOLATION AND DETECTION OF SPECIFIC ANTIBODIES FROM SERUM", Conference Proceedings - NANOCON 2015, 1 January 2015 (2015-01-01), XP093391144, Retrieved from the Internet: URL:https://www.confer.cz/nanocon/2015/download/639-on-chip-elisa-on-magnetic-particles-isolation-and-detection-of-specific-antibodies-from-serum.pdf<br>* abstract ; Table 1 ; pg 478, para 2.3 *<br>----- | 10,11<br><br>1-9 | INV.<br>G01N33/543 |
| X<br><br>Y | IDIL NESLIHAN ET AL: "Concanavalin A immobilized magnetic poly(glycidyl methacrylate) beads for prostate specific antigen binding", COLLOIDS AND SURFACES B: BIOINTERFACES ELSEVIER AMSTERDAM, NL, vol. 134, 4 July 2015 (2015-07-04), pages 461-468, XP029269680, ISSN: 0927-7765, DOI: 10.1016/J.COLSURFB.2015.06.050<br>* abstract ; Fig 1c *<br>----- | 10,11<br><br>1-9 | TECHNICAL FIELDS SEARCHED (IPC)<br>G01N |
| X | HORáK DANIEL ET AL: "Albumin-coated monodisperse magnetic poly(glycidyl methacrylate) microspheres with immobilized antibodies: Application to the capture of epithelial cancer cells", JOURNAL OF BIOMEDICAL MATERIALS RESEARCH PART A, vol. 101A, no. 1, 6 July 2012 (2012-07-06), pages 23-32, XP055846865, US<br>ISSN: 1549-3296, DOI: 10.1002/jbm.a.34297<br>* abstract ; Fig 6 *<br>-----<br>-/-- | 10,11 | |

The present search report has been drawn up for all claims

1

| Place of search | Date of completion of the search | Examiner |
|---|---|---|
| The Hague | 21 May 2026 | Vadot-Van Geldre, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 25 22 3430

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HUERGO LUCIANO F. ET AL: "Magnetic Bead-Based Immunoassay Allows Rapid, Inexpensive, and Quantitative Detection of Human SARS-CoV-2 Antibodies", ACS SENSORS, vol. 6, no. 3, 26 January 2021 (2021-01-26), pages 703-708, XP093391728, US ISSN: 2379-3694, DOI: 10.1021/acssensors.0c02544 * abstract ; Fig 1-2 ; pg 704, col 1, para 2 ; pg 706, col 2, para 1 * ----- | 1-9 | |
| | | | TECHNICAL FIELDS SEARCHED (IPC) |

The present search report has been drawn up for all claims

1

| Place of search | Date of completion of the search | Examiner |
|---|---|---|
| The Hague | 21 May 2026 | Vadot-Van Geldre, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *CHEMICAL ABSTRACTS*, 3945-69-5